# EUROPEAN PATENT APPLICATION

(11) **EP 4 650 163 A1**
(43) Date of publication of application: **19.11.2025**
(21) Application number: 24176059.4
(22) Date of filing: 15.05.2024
(51) Int. Cl.: B32B 5/02, B32B 5/18, B32B 5/24, B32B 7/12, B32B 9/02, B32B 9/04, C12N 1/14, D06N 3/00

(54) **MYCELIUM COMPOSITE MATERIAL**

(71) Applicant: Vrije Universiteit Brussel, 1050 Brussel (BE)
(72) Inventor: VANDELOOK, Simon, 1050 Ixelles (BE); PEETERS, Eveline, 3150 Haacht (BE)
(74) Representative: Winger

(57) **Abstract**

In a first aspect, the present invention relates to a composite material (10), comprising: i) a layer of mycelium (20), ii) a layer of binder (30), and iii) a backing (40); wherein the binder (30) comprises a thermoreversible gel.

## Description

### Technical field of the invention

The present invention relates to composite materials, and more in particular to the development and fabrication of composite materials with the aim of creating a sustainable leather-like material.

### Background of the invention

In the realm of material science, there has been a growing interest in the development of bio-based materials that can offer similar characteristics to those of animal leather. Mycelium, the vegetative part of a fungus, has emerged as a promising candidate in this regard. Mycelium-based materials, often referred to as "mushroom leather," have the potential to revolutionize the leather industry by providing a more sustainable and eco-friendlier alternative. The technological progress with these materials was recently reviewed by Elsacker et al. (Elsacker, E., Vandelook, S., & Peeters, E. (2023). Recent technological innovations in mycelium materials as leather substitutes: a patent review. Frontiers in Bioengineering and Biotechnology, 11.)

The general problem currently faced in the field of mycelium technology is the challenge of producing mycelium-based materials that possess the necessary strength and durability to function as viable leather substitutes. While mycelium skins produced through fermentation processes can mimic the appearance and texture of leather, they often lack the requisite mechanical properties, such as tensile strength, to withstand the wear and tear associated with everyday use. With values falling within the range of 0.27 to 1.5 MPa, the tensile strength of untreated mycelium mats sourced from surface fermentation processes is significantly lower than that of genuine leather or its synthetic counterparts, which limits their practical application.

Thus far, focus has been mostly placed on enhancing mechanical properties of the mycelium by using crosslinking strategies: in this way, chemical modifications enable to improve the material's strength while maintaining its flexibility. However, such crosslinking strategies may compromise the biodegradability and the bio-based nature of the material. More in particular, while various crosslinking agents and methods have been explored to improve the strength and flexibility of mycelium-based materials, these approaches often introduce non-biodegradable components or require complex processing methods that do not align with the sustainability goals of using mycelium as a base material.

Alternatively, the integration of mycelium with other materials to create composites has been considered as a means to improve mechanical properties. However, the challenge remains to find suitable materials and methods that can reinforce mycelium without detracting from its environmental benefits.

Despite the advancements made in the field and the various strategies employed to enhance the properties of mycelium-based materials, there is still a need for further innovation. Accordingly, the development of new materials and processes that can address the limitations of current mycelium-based materials remains essential for realizing their full potential as sustainable alternatives to traditional leather.

### Summary of the invention

It is an object of the present invention to provide good mycelium-based composite materials. It is a further object of the present invention to provide methods and uses associated therewith. This objective is accomplished by composite materials, methods and uses according to the present invention.

It was surprisingly found within the present invention that a composite material with good mechanical properties and a natural, leather-like appearance and texture-thereby yielding a robust, flexible, and eco-friendly alternative to traditional leather-can be formed by binding a mycelium layer to a backing through a thermoreversible gel. More in particular, the mycelium provides the natural, leather-like appearance on the outside (at one or both sides of the composite material; cf. infra); the backing improves the mechanical properties of the mycelium; and the thermoreversible gel binder forms a strong bond to and between the backing and mycelium (e.g. including (partially) penetrating into the backing and/or mycelium), while also acting as a levelling agent that filled voids or uneven surfaces and even providing additional mechanical advantages (e.g. flexibility/pliability) to the composite material.

In a first aspect, the present invention relates to a composite material, comprising: i) a layer of mycelium, ii) a layer of binder, and iii) a backing; wherein the binder comprises a thermoreversible gel.

In a second aspect, the present invention relates to a product comprising the composite material according to any embodiment of the first aspect.

In a third aspect, the present invention relates to a method for forming a composite material, comprising: a) providing a stack of a layer of mycelium, and either a layer of binder and a backing or a backing infused with a binder; and b) binding the mycelium to the backing using the binder.

In a fourth aspect, the present invention relates to a use of a layer of binder in a composite material as defined in any embodiment of the first aspect, for binding the mycelium to the backing.

It is an advantage of embodiments of the present invention that an alternative-with similar characteristics-to animal leather and/or synthetic leathers can be formulated, which is more environmentally friendly. It is a further advantage of embodiments of the present invention that the components for the composite material can be source from renewable and/or durable sources.

It is an advantage of embodiments of the present invention that a composite material can be created with a layer of mycelium that mimics the texture and appearance of traditional leather.

It is an advantage of embodiments of the present invention that the binder used in the composite material is a thermoreversible gel, which allows for reversible transitions between gel and liquid states under controlled temperature conditions, facilitating the manufacturing, binding-and optionally even repairing-of the composite material, and allowing to form a strong, durable bond between the mycelium and backing.

It is an advantage of embodiments of the present invention that the backing provides structural integrity and contributes to the mechanical strength of the composite material.

It is an advantage of embodiments of the present invention that the binder and/or mycelium can include a plasticizing agent, which can improve their individual flexibility and durability, as well as that of the composite material as a whole.

It is an advantage of embodiments of the present invention that the mycelium, binder, and backing can independently include one or more additives, allowing for customization of the material's properties to meet specific requirements such as colour, texture or additional functionalities.

It an advantage of embodiments of the present invention that the composite material can produced with a leather-like mycelium layer on one or both sides; e.g. depending on the overall performance and aesthetic appeal desired for a certain application.

It is an advantage of embodiments of the present invention that the composite material can be manufactured in a relatively straightforward, scalable and economic fashion.

Particular and preferred aspects of the invention are set out in the accompanying independent and dependent claims. Features from the dependent claims may be combined with features of the independent claims and with features of other dependent claims as appropriate and not merely as explicitly set out in the claims.

Although there has been constant improvement, change and evolution of devices in this field, the present concepts are believed to represent substantial new and novel improvements, including departures from prior practices, resulting in the provision of more efficient, stable and reliable devices of this nature.

The above and other characteristics, features and advantages of the present invention will become apparent from the following detailed description, taken in conjunction with the accompanying drawings, which illustrate, by way of example, the principles of the invention. This description is given for the sake of example only, without limiting the scope of the invention. The reference figures quoted below refer to the attached drawings.

### Brief description of the drawings

FIG 1 is a schematic representation of the production process of mycelium skins for the composite material according to illustrative embodiments of the present invention, showing the provision of an inoculum (left), homogenizing the inoculum with a liquid medium (centre) and incubating this in a container (right).
FIG 2 and FIG 3 are photographs of mycelium skins being grown in metal catering containers according to an illustrative embodiment of the present invention.
FIG 4 is a photograph of a dried mycelium skin according to an illustrative embodiment of the present invention.
FIG 5 is a scanning electron microscope (SEM) image of a mycellium skin coated with a Lotus binding agent.
FIG 6 is a graph of water contact angle measurements (in °C) of the aerial (left) and wet (right) side of mycelium skins according to an illustrative embodiment of the present invention. The axis on the left indicates the hydrophilic (0-89 °C), hydrophobic (90-149 °C) and super-hydrophobic (150-180 °C) zones.
FIG 7 is a schematic representation of the production process of a gelatine-based hydrogel sheet for the composite material according to illustrative embodiments of the present invention, showing dissolving and mixing the hydrogel (top left), adding and mixing in optional further compounds (top right), pouring the mixture into a mould (bottom left), drying and solidifying it (bottom middle) and obtaining hydrogel sheets (bottom right).
FIG 8 is a photograph of a 'dried' gelatine-based hydrogel sheet according to an illustrative embodiment of the present invention.
FIG 9 schematically depicts a stack for forming a sandwich-structured composite material according to illustrative embodiments of the present invention.
FIG 10 is a photograph of an assembled composite material according to an illustrative embodiment of the present invention.
FIG 11 is a photograph of a metal grid insert with a diamond pattern in accordance with an illustrative embodiment of the present invention.
FIG 12 is a photograph of a mycelium composite material embossed with the insert of FIG 11 according to an illustrative embodiment of the present invention.
FIG 13 is a photograph of a metal plate insert with a rough plaster relief in accordance with an illustrative embodiment of the present invention.
FIG 14 is a photograph of a mycelium composite material embossed with the insert of FIG 13 according to an illustrative embodiment of the present invention.
FIG 15 shows electron microscopy images of a sandwich-structured composite material according to an illustrative embodiment of the present invention.
FIG 16 is a graph of the ultimate stress (in MPa) of different composite materials according to illustrative embodiments of the present invention (a: jute, b: fine linen, c: heavy linen, d: fine hemp, and e: large hemp) compared to PU (f), PUV (g) and cowhide leather (h) as benchmark materials.
FIG 17 is a graph of the ultimate strain (in %) of different composite materials according to illustrative embodiments of the present invention (a: jute, b: fine linen, c: heavy linen, d: fine hemp, and e: large hemp) compared to PU (f), PUV (g) and cowhide leather (h) as benchmark materials.
FIG 18 is a graph of the Young's modulus (in MPa) of different composite materials according to illustrative embodiments of the present invention (a: jute, b: fine linen, c: heavy linen, d: fine hemp, and e: large hemp) compared to PU (f), PUV (g) and cowhide leather (h) as benchmark materials.
FIG 19 is a graph of the ultimate force (in N) of different composite materials (black) according to illustrative embodiments of the present invention (a: jute, b: fine linen, c: heavy linen, d: fine hemp, and e: large hemp) compared to their corresponding naked backings (white).
FIG 20 is a plot into an Ashby material chart of the tested composite materials according to illustrative embodiments of the present invention (3: heavy linen, fine and large hemp and jute, and 4: fine linen) and benchmark materials (1: PU and PUV, 2: leather).

In the different figures, the same reference signs refer to the same or analogous elements.

### Description of illustrative embodiments

The present invention will be described with respect to particular embodiments and with reference to certain drawings, but the invention is not limited thereto but only by the claims. The drawings described are only schematic and are non-limiting. In the drawings, the size of some of the elements may be exaggerated and not drawn on scale for illustrative purposes. The dimensions and the relative dimensions do not correspond to actual reductions to practice of the invention.

Furthermore, the terms first, second, third and the like in the description and in the claims, are used for distinguishing between similar elements and not necessarily for describing a sequence, either temporally, spatially, in ranking or in any other manner. It is to be understood that the terms so used are interchangeable under appropriate circumstances and that the embodiments of the invention described herein are capable of operation in other sequences than described or illustrated herein.

Moreover, the terms top, bottom, over, under and the like in the description and the claims are used for descriptive purposes and not necessarily for describing relative positions. It is to be understood that the terms so used are interchangeable with their antonyms under appropriate circumstances and that the embodiments of the invention described herein are capable of operation in other orientations than described or illustrated herein.

It is to be noticed that the term "comprising", used in the claims, should not be interpreted as being restricted to the means listed thereafter; it does not exclude other elements or steps. It is thus to be interpreted as specifying the presence of the stated features, integers, steps or components as referred to, but does not preclude the presence or addition of one or more other features, integers, steps or components, or groups thereof. The term "comprising" therefore covers the situation where only the stated features are present and the situation where these features and one or more other features are present. Thus, the scope of the expression "a device comprising means A and B" should not be interpreted as being limited to devices consisting only of components A and B. It means that with respect to the present invention, the only relevant components of the device are A and B.

Similarly, it is to be noticed that the term "coupled", also used in the claims, should not be interpreted as being restricted to direct connections only. The terms "coupled" and "connected", along with their derivatives, may be used. It should be understood that these terms are not intended as synonyms for each other. Thus, the scope of the expression "a device A coupled to a device B" should not be limited to devices or systems wherein an output of device A is directly connected to an input of device B. It means that there exists a path between an output of A and an input of B which may be a path including other devices or means. "Coupled" may mean that two or more elements are either in direct physical or electrical contact, or that two or more elements are not in direct contact with each other but yet still co-operate or interact with each other.

Reference throughout this specification to "one embodiment" or "an embodiment" means that a particular feature, structure or characteristic described in connection with the embodiment is included in at least one embodiment of the present invention. Thus, appearances of the phrases "in one embodiment" or "in an embodiment" in various places throughout this specification are not necessarily all referring to the same embodiment, but may. Furthermore, the particular features, structures or characteristics may be combined in any suitable manner, as would be apparent to one of ordinary skill in the art from this disclosure, in one or more embodiments.

Similarly, it should be appreciated that in the description of exemplary embodiments of the invention, various features of the invention are sometimes grouped together in a single embodiment, figure, or description thereof for the purpose of streamlining the disclosure and aiding in the understanding of one or more of the various inventive aspects. This method of disclosure, however, is not to be interpreted as reflecting an intention that the claimed invention requires more features than are expressly recited in each claim. Rather, as the following claims reflect, inventive aspects lie in less than all features of a single foregoing disclosed embodiment. Thus, the claims following the detailed description are hereby expressly incorporated into this detailed description, with each claim standing on its own as a separate embodiment of this invention.

Furthermore, while some embodiments described herein include some but not other features included in other embodiments, combinations of features of different embodiments are meant to be within the scope of the invention, and form different embodiments, as would be understood by those in the art. For example, in the following claims, any of the claimed embodiments can be used in any combination.

In the description provided herein, numerous specific details are set forth. However, it is understood that embodiments of the invention may be practised without these specific details. In other instances, well-known methods, structures and techniques have not been shown in detail in order not to obscure an understanding of this description.

The following terms are provided solely to aid in the understanding of the invention.

As used herein, and unless otherwise specified, the term "composite material" refers to an engineered material made from two or more constituent materials with significantly different physical or chemical properties that, when combined, produce a material with characteristics different from the individual components.

As used herein, and unless otherwise specified, the term "mycelium" refers to the vegetative (root-like) part of a fungus, consisting of a network of fine filaments (hyphae). The mycelium as used in the present invention may be derived from various fungal species; typically filamentous fungal species like a xylophagous fungus (e.g. a white-rot fungus). Specific examples include a basidiomycete (e.g. *Ganoderma* spp., *Trametes* spp., *Fomes* spp., *Schizophyllum* spp. and *Pleurotus* spp.) or an ascomycete (e.g. *Fusarium* spp.). The mycelium as used in the present invention is preferably a dikaryon. A layer of mycelium is herein also referred to as a "mycelium skin" or "mycelium mat". The above fungi are generally able to degrade a wide variety of organic substrates-including difficult-to-degrade wood-containing lignocellulosic substrates-, and are therefore advantageously be grown on agricultural and/or industrial waste or side streams, giving rise to an interesting class of natural materials with (highly) tuneable properties.

As used herein, and unless otherwise specified, the term "binder" refers to a substance that holds or binds (e.g. glues) other materials together to form a cohesive whole. Within the present invention, the binder comprises (e.g. consists of) a thermoreversible gel.

As used herein, and unless otherwise specified, the term "gel" refers to a semi-solid, and more specifically-as defined by the International Union of Pure and Applied Chemistry (IUPAC)-: a nonfluid colloidal network or polymer network that is expanded throughout its whole volume by a fluid. A gel in which the fluid (also referred to as "swelling agent") is a water is referred to as a "hydrogel". An open network formed by the removal of all swelling agents from a gel is referred to as a "xerogel".

As used herein, and unless otherwise specified, the term "thermoreversible gel" refers to a gel that can transition between the gel state and a liquid state upon changes in temperature, specifically being able to gel upon cooling and liquefy upon heating. Without being bound by theory, this transition is typically possible because the junction points in the gel network (i.e. colloidal or polymer network) are thermally reversible. A thermoreversible gel is typically characterized by having a "gel temperature" (also referred to as "gelation temperature"), which is the temperature at which there is an abrupt change in the viscosity-and fluidity-of the substance, and below which the gel state is formed. A thermoreversible gel may further have a melting temperature, which is the temperature above which the substance-and in particular its viscosity-behaves like a classical solution. In between both, the substance is then typically in intermediate regime, where the viscosity transitions from one behaviour to the next.

As used herein, and unless otherwise specified, the term "gelatinous substance" refers to a substance comprising gelatine or a similar gelling agent-such as a protein (e.g. whey protein, casein or soy protein), a (natural) gum (e.g. agar, carrageenan or Gellan gum), pectin or starch.

As used herein, and unless otherwise specified, the term "backing" refers to a layer of material used to provide support and/or stability to the composite material.

As used herein, and unless otherwise specified, the term "mesh" refers to a porous material made from interconnected strands (e.g. filaments) with regularly or irregularly spaced holes.

As used herein, and unless otherwise specified, the term "fabric" refers to a textile material which may be woven, non-woven (bonded), felted, knitted, stitched, crocheted, knotted, tatted, braided, etc.

As used herein, and unless otherwise specified, the term "natural fibres" refers to fibres derived from plants, animals or minerals.

As used herein, and unless otherwise specified, the term "plasticizing agent" (or "plasticizer") refers to a substance added to a material to make it more flexible. In embodiments, such a plasticizing agent may be (or at least comprise) a humectant (i.e. a hygroscopic substance which attracts and holds water to keep moisture in the material it is added to); thereby aiding to keep in particular the mycelium and binder-but optionally also the backing-sufficiently moist. Mycelium and/or binder (and/or backing) that has dried out too much (i.e. having a low moisture content, e.g. below about 20 wt%) can typically become stiff and brittle, and might crack when pulled or bent. Notwithstanding, also non-humectant plasticizers can be used.

As used herein, and unless otherwise specified, the term "additive" refers to a substance added to a material to modify its properties or to confer specific characteristics. Specific embodiments of additive may include, but are not limited to, a dyeing agent, antimicrobial agent, antioxidant, pH-modifier or a crosslinking agent.

As used herein, and unless otherwise specified, the term "sandwich-structured composite material" refers to a composite material in which central layer (herein the backing) is provided between two outer layers (herein two layers of mycelium, each bound to the backing by a layer of binder). The outer layers thereby clad two opposing sides of the central layer.

Unless otherwise specified, all actions described herein may be performed at normal temperature (i.e. 20 °C) and pressure (i.e. 1 atm).

In a first aspect, the present invention relates to a composite material, comprising: i) a layer of mycelium, ii) a layer of binder, and iii) a backing; wherein the binder comprises a thermoreversible gel.

Most typically, the binder may directly bind the layer of mycelium to the backing. Notwithstanding, in some embodiments the binder may indirectly bind the layer of mycelium to the backing. For example, the composite material may optionally comprise one or more additional layers (including additional binding layers and/or backing layers) and the layer of binder may indirectly bind the layer of mycelium to the backing in the sense that it binds two (or more) layers of the stack together but does not as such directly bind the layer of mycelium to the backing. In such a case, the rest of stack may for instance be held together by additional connecting means (e.g. one or more additional layers of binder or any other suitable means, such as stitching, touch-fastening, riveting, etc.)

In embodiments, the thermoreversible gel may be a hydrogel. A hydrogel may advantageously be relatively easy to produce. Moreover, it can retain a significant amount of water (i.e. its swelling agent), potentially improving the flexibility, workability and durability of the composite material (in particular the mycelium). In embodiments, the thermoreversible gel may comprise (e.g. be) a gelatinous substance. In preferred embodiments, the gelatinous substance may be a protein-based gelatinous substance, preferably gelatine. Protein-based gelatinous substance can advantageously provide a strong adhesive bond between mycelium layer and backing. Although most gelatine is sourced from an animal-based origin (which may be less preferred from a sustainability point-of-view), it is advantageously a biodegradable and non-toxic substance, which forms an abundant side stream of animal production and processing (so that the use thereof can be regarded as a form of waste reduction, and thus nevertheless an asset in terms of sustainability). Additionally, non-animal-based methods (applicable on industrial scale) for producing gelatine exist; such as through heterologous expression in microorganism (e.g. yeast), to name but one example.

In embodiments, the thermoreversible gel may have a gel temperature (e.g. measured at normal pressure), below 200 °C, preferably below 180 °C, more preferably below 150 °C, yet more preferably below 120 °C, still yet more preferably below 100 °C, most preferably below 80 °C. A lower gel temperature advantageously allows the thermoreversible gel to be fluidified ('molten')-and thus processed-at lower temperatures, which is energy-efficient and can prevent damage to the other components of the composite material (in particular the mycelium). Notwithstanding, a somewhat higher gel temperature (e.g. above 100 °C or higher) may in some applications be preferred so that the composite material can withstand (i.e. doesn't degrade under) such temperatures, thereby improving its lifespan.

In embodiments, the backing may be a mesh. A mesh backing can advantageously provide structural integrity and support to the composite material while allowing for flexibility and a good-at least partial-penetration of the binder into the mesh, thereby improving its binding therewith. In embodiments, the mesh may be a fabric made from natural fibres, preferably plant fibres (e.g. hemp, cotton, jute, flax, ramie, kenaf, coir, bamboo or linen) or animal fibres (e.g. wool or silk), most preferably plant fibres. In embodiments, the fabric made from plant fibres may be a cellulosic (i.e. predominantly composed of a cellulose material) or lignocellulosic (i.e. predominantly composed of a material comprising lignin cellulose and hemicellulose) fabric. Plant and animal fibres are advantageously typically renewable and biodegradable, and-in particular plant fibres-can be sourced in a sustainable manner.

In embodiments, the binder may comprise a plasticizing agent. In embodiments, the volume ratio of plasticizing agent to binder (plasticizing agent:binder) may from 2:1 to 1:2, preferably from 3:2 to 2:3, more preferably from 4:3 to 3:4, still more preferably from 5:4 to 4:5, most preferably 1:1. In embodiments, the mycelium may comprise a plasticizing agent (e.g. the mycelium may have been contacted with-e.g. immersed in-the plasticizing agent prior to contacting it with the binder). In embodiments, the mycelium may comprise 5-35% v/v of the plasticizing agent, preferably 10-30% v/v, most preferably 15-25% v/v; such as 20% v/v. The inclusion of a plasticizing agent advantageously enhances the flexibility and workability of the mycelium and/or binder-and to a lesser extent also the backing-, leading to a more pliable composite material which is less prone to cracking. Since in particular both the binder and mycelium are prone to stiffness and brittleness (e.g. at low moisture levels), preferably both the binder and the mycelium may comprise (an independently selected) plasticizing agent (which may be a humectant or non-humectant plasticizer). Notwithstanding, since in the composite material the binder may typically (at least partially) penetrate the mycelium (and backing), it may in some embodiments suffice to include as such a plasticizing agent in either the binder or the mycelium (e.g. only in the binder), which can then have a plasticizing effect on the mycelium and binder (and backing) in the final composite material. In embodiments, the plasticizing agent may be a polyol (e.g. propylene glycol or glycerol), castor oil, sugar alcohol, epoxy ester, ester plasticizer, glycerol ester, phosphate ester, terephthalate, leather conditioner, acetylated monoglyceride, alkyl citrate, epoxidized vegetable oil, methyl ricinolate or another polymer plasticizer. In preferred embodiments, the plasticizing agent may be glycerol. Glycerol is advantageously commonly used in food applications as a humectant and is thus a safe and readily available plasticizing agent.

In embodiments, the layer of mycelium may have a length (e.g. a longest dimension) of at least 5 cm, preferably at least 10 cm, more preferably at least 20 cm, still more preferably at least 30 cm, yet still more preferably at least 50 cm, most preferably at least 60 cm, such as at least 1.0 m or 1.5 m. In embodiments, the layer of mycelium may have a width (perpendicular to the length) of at least 5 cm, preferably at least 10 cm, more preferably at least 20 cm, still more preferably at least 30 cm, yet still more preferably at least 50 cm, most preferably at least 60 cm, such as at least 1.0 m or 1.5 m. In embodiments, the layer of mycelium may have an area of at least 25 cm², preferably at least 100 cm², more preferably at least 400 cm², still more preferably at least 900 cm², yet still more preferably at least 2500 cm², most preferably at least 3600 cm², such as at least 1 m² or 1.5 m². In preferred embodiments, the layer of mycelium may comprise (e.g. may be) a continuous mycelium network of the aforementioned dimensions.

In embodiments, the layer of mycelium may be derived from a *Ganoderma* sp., *Trametes* sp., *Fomes* sp., *Schizophyllum* sp., *Pleurotus* sp. or *Fusarium* sp., preferably *Ganoderma* sp. or *Trametes* sp., most preferably *Trametes versicolor.* The latter in particular were advantageously observed to have superior performance properties (e.g. denser mycelium) compared to mycelium derived from other fungi.

In embodiments, the mycelium, binder and/or backing may independently further comprise one or more additives. Additives can advantageously be used to tailor the properties-such as colour, strength, or resistance to environmental factors-of the composite material. For instance, the mycelium may be coated with a binding agent (e.g. OrganoClick OC-BioBinder^{™} Lotus 54)-not to be confused with the binder-for binding the hyphae and coating the mycelium. Such a binding agent may for example be applied to the mycelium as part of a post-treatment (e.g. by means of submersion in a treatment bath or applied directly using a paint bursh or similar application means) after harvesting but before including the mycelium into the composite material. It was advantageously found that-without being bound by theory: through binding the hyphae and coating the myceliumsuch binding agents can significantly improve the mycelium's properties, such as its flexibility (i.e. akin to a plasticizing agent), hydrophobic properties, Taber abrasion resistance and wet surface abrasion resistance. Moreover, the binding agent could double up as a dyeing agent, with different shades of brown being available depending on the length (e.g. 1 hour to 100 hours) and temperature (e.g. 60-150 °C, preferably 80 to 100 °C) of curing (e.g. using an oven or heat press).

In embodiments, the composite material may be a sandwich-structured composite material comprising: i) a first layer of mycelium, ii) a first layer of binder, iii) a backing, iv) a second layer of binder, and v) a second layer of mycelium. A sandwich-structure advantageously allows to have a composite material with good mechanical properties while having a natural, leather-like appearance and texture on both sides of the material.

In embodiments, any feature of any embodiment of the first aspect may independently be as correspondingly described for any embodiment of any of the other aspects.

In a second aspect, the present invention relates to a product comprising the composite material according to any embodiment of the first aspect.

Such a product may generally be any product wherein leather-like materials can be used- or even products wherein such materials are not (yet) used-, including but not limited to: a bag (e.g. handbag or carry on), seating and/or cushioning (e.g. a couch, chair or seat), finishing (e.g. for a car interior), footwear (e.g. shoes), etc.

In embodiments, any feature of any embodiment of the second aspect may independently be as correspondingly described for any embodiment of any of the other aspects.

In a third aspect, the present invention relates to a method for forming a composite material, comprising: a) providing a stack of a layer of mycelium, and either a layer of binder and a backing or a backing infused with a binder; and b) binding the mycelium to the backing using the binder.

In embodiments, providing the stack of mycelium may comprise forming the mycelium by fermentation, such as a liquid-state (LSF) or solid-state (SSF) fermentation. In preferred embodiments, the fermentation may be surface fermentation, such as a liquid-state (LSSF) or solid-state (SSSF) surface fermentation; preferably LSSF. Surface fermentation like LSSF or SSSF advantageously allow to grow the fungi without agitation or homogenisation, thereby enabling a higher degree of crosslinking within the mycelium network and thereby forming a continuous mycelium sheet. LSSF may moreover be preferred over SSSF because it allows: 1) more versatility in the feedstocks used (including both liquid organic and submerged solid feedstocks, compared to solid feedstocks only for SSSF); 2) easy separation after growth of the buoyant mycelium from the liquid feedstock (compared to needing additional separation membranes over the solid feedstock in order to be able to remove the mycelium from the feedstock); 3) optimal enzyme dispersion in the liquid medium for efficient catabolism; 4) facile recovery of valuable fermentation by-products such as exopolysaccharides, enzymes and/or metabolites; 5) easy preparation and piping of reagents and feedstocks, facilitating continuous processing. Notwithstanding, the fermentation may in other embodiments be a stirred submerged liquid fermentation (SSLF). Compared to surface fermentation, SSLF typically involves agitation and/or homogenisation, thereby yielding a less cohesive broth and accordingly non-continuous mycelium sheets. In embodiments, providing the stack of mycelium may comprise partially drying (i.e. without drying out) the mycelium. For instance, the mycelium may have an initial weight ('wet weight') upon harvesting and may be dried so that its weight after drying is between 10 and 90% of the initial weight, preferably between 20 and 70%, more preferably between 30 and 50%. This can for example be performed in a well-ventilated space at room temperature (e.g. around 20 °C) or in a ventilated oven at 40-80 °C; e.g. for a duration of 2 to 24 hours. In embodiments, the layer of mycelium may have a more hydrophobic side (e.g. the aerial side of a liquid-state surface fermentation) and a more hydrophilic side (e.g. the liquid side of a liquid-state surface fermentation), and the stack may be provided such that the hydrophobic side of the layer of mycelium face outwards (i.e. the hydrophilic side faces towards the layer of binder). The binder may typically be hydrophilic and thus advantageously more compatible with the hydrophilic side of the mycelium, while having the hydrophobic side face outwards can additionally be advantages in giving the composite material a water-repellent and thereby more dirt/stain-resistant exterior.

In embodiments, the mycelium and/or binder-preferably both-provided in step a may comprise a plasticizing agent.

In embodiments, step a may comprise providing the binder as a solid gel. For example, this may comprise 'drying' (but not so much as to form an actual xerogel, i.e. leaving at least some fluid in the gel) the binder so as to form the solid gel. This advantageously simplifies the handling of the binder during step a, compared to a wet or fluidified gel. In some embodiments, step a may comprise providing the layer of binder as a solid layer (i.e. separate from the backing; e.g. a sheet). This advantageously allows to handle the binder in this step as a discrete layer, which can be precisely placed and controlled. In other embodiments, step a may comprise providing the backing infused with the binder. This advantageously allows to reduce the number of layers that need to be manipulated in this step, as the binder and backing are then handled as one.

In embodiments, step b may comprise: b1) heating the stack such that the binder becomes fluid, and b2) cooling the stack to re-gel the binder. By binding the mycelium to the backing through first fluidifying the binder and re-gelling it, the binder can advantageously (partially) penetrate into the backing and/or mycelium, thereby forming a strong bond to and between the backing and mycelium, ensuring a secure lamination. In embodiments, heating the stack such that the binder becomes fluid may comprise heating the binder above its gel point, preferably above its melting point. In embodiments, step b1 may be performed at temperature of 200 °C or below, preferably 180 °C or below, more preferably 150 °C or below, yet more preferably 120 °C or below, still yet more preferably 100 °C or below. Gelatine binder sheets (e.g. as described in the Example below) for instance typically rapidly (e.g. within 10 s) fluidify ('melt') at temperatures of 100 °C or above. A lower heating temperature-but nevertheless sufficient to fluidify the binder in a relatively brief time-are advantageously energy-efficient and can prevent damage to the other components of the composite material. While the other components may often (momentarily) take relatively elevated temperatures (e.g. above 180-200 °C) without apparent degradation, they-in particular the mycelium-can typically not do so for prolonged periods (e.g. the mycelium may become brown and smell 'cooked'). Accordingly, step b1 may in preferred embodiments be performed for 15 min or less, preferably 10 min or less, more preferably 5 min or less, still more preferably 150 s or less, most preferably 120 seconds or less; such as 10 to 120 seconds, e.g. 20 to 40 s, 40 to 60 s, 60 to 90 s or 90 to 120 s.

In embodiments, step b may be performed while pressing the stack. Step b1 (and b2) may for instance be preformed in a heat press. Pressing the stack can advantageously help to ensure a uniform bond, as well as eliminate any voids (e.g. air pockets) or unevenness between layers. A flat pressing surface (e.g. in contact with the mycelium layer) may be used to create a smooth surface (e.g. a smooth mycelium outer layer). Alternatively, the composite material (in particular the mycelium layer) can be embossed (i.e. provided with a texture) by using a patterned pressing surface (e.g. in the form of a pressing device with a patterned surface or a patterned insert placed between the pressing device and the stack).

In embodiments, the method may be for forming the sandwich-structured composite material as defined above. In such embodiments, step a may comprise: a) providing a stack of a first layer of mycelium, either a first layer of binder, a backing, and a second layer of binder; or a backing infused with a binder, and a second layer of mycelium. A sandwich-structure advantageously allows to have a composite material with good mechanical properties while having a natural, leather-like appearance and texture on both sides of the material.

In embodiments, any feature of any embodiment of the third aspect may independently be as correspondingly described for any embodiment of any of the other aspects.

In a fourth aspect, the present invention relates to a use of a layer of binder in a composite material as defined in any embodiment of the first aspect, for binding the mycelium to the backing.

In embodiments, any feature of any embodiment of the fourth aspect may independently be as correspondingly described for any embodiment of any of the other aspects.

The invention will now be described by a detailed description of several embodiments of the invention. It is clear that other embodiments of the invention can be configured according to the knowledge of the person skilled in the art without departing from the true technical teaching of the invention, the invention being limited only by the terms of the appended claims.

### Example

In this example, the aim was to manufacture a composite material possessing leather-like attributes while being entirely constituted of bio-based and sustainable organic components. The resulting material had a sandwich-like composition, with mycological tissue on both outer surfaces to replicate a leather-like texture (although a structure with only one mycelium layer-e.g. for applications where only one outer surface is exposed/accessible-was also achievable). The sandwich's core was a plant- or animal-based mesh-providing mechanical strength-,while a gelatine-based hydrogel functioned as a binder that connected the first two elements. The final composite material exhibited flexibility and a robustness and appearance akin to animal skin leather.

### Mycelium skins

Mycelium skins formed the outside, visible layers of the composite material and were therefore crucial in determining the sensorial (look-and-feel) characteristics of the product. Mycelium skins were attainable from diverse genera of filamentous fungi such as *Ganoderma* spp., *Trametes* spp., *Fomes* spp., *Schizophyllum* spp., and *Pleurotus* spp. These were typically white-rot fungi belonging to Basidomycota. All species were dikaryons.

Strains were maintained at 4 °C on agar plates with minimal medium (MM). Strains were cultured at 28 °C in the dark for 7 days on agar plates with complete yeast medium (CYM: 2% glucose, 0.3% peptone, 0.2% yeast extract, 0.05% MgSO₄, pH 6), or a medium of grinded malt grains or wheat grains (30 g/L) and 1.5% corn steep liquor. Liquid cultures were prepared in autoclavable microbox containers (e.g. 90 mm diameter, 360 mL volume capacity) or metal catering containers (e.g. size GN1/1) fitted with gas exchange filters purchased at Sac O₂ NV, Veldeken 38 b, 8950 Deinze (Belgium). Inoculum was prepared by blending half a fully grown petri dish/L medium for 6-8 s at low-medium speed in a semi-micro blender (Waring) containing 100 mL medium (e.g. CYM). Cultures were supplemented with Streptomycin (25 µg/L) to avoid bacterial contamination. Alternatively, the inoculum could also be prepared from a spore solution.

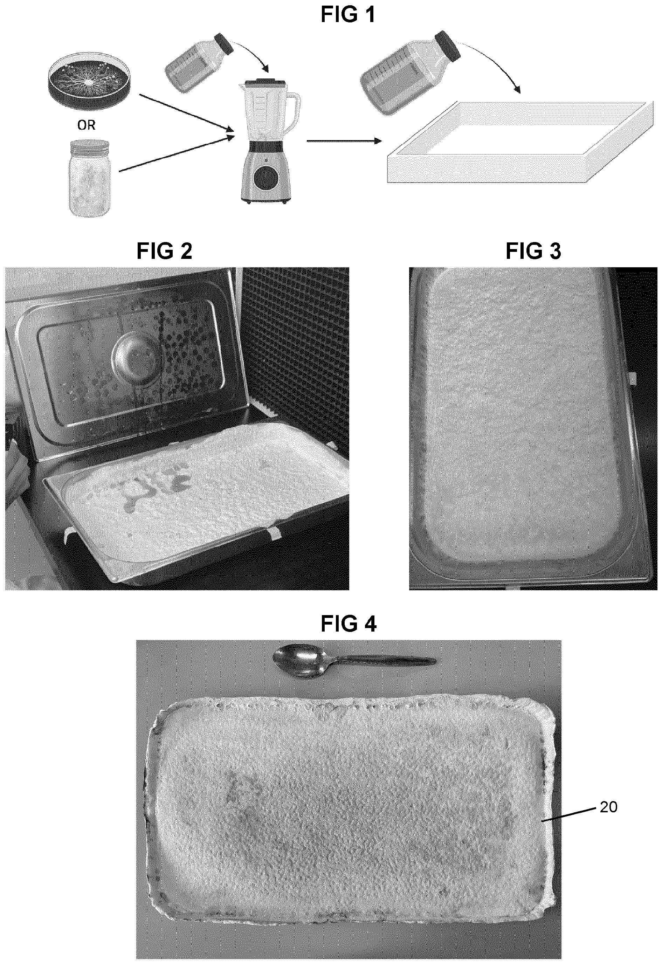

Mycelium skins were in this example produced using a liquid-state surface fermentation (LSSF) approach (FIG 1). Initiating mycelium mat cultures involved homogenizing a fully grown agar-based starter culture or a spore solution with a small volume of approximately 100 mL of liquid medium or water. Effective homogenization was achieved using a semimicro blender like Waring within 6-8 seconds of low to medium speed blending. It was noted that a higher blending speed or time could lead to excessive shearing of hyphae, thereby negatively impacting fungus viability. Next, part of this inoculum was added (25-50 mL) to a larger volume of a liquid nutrient-rich medium containing a combination of a carbon source, protein source, and a source of essential micro-nutrients in a solid or liquid form (e.g. any type of lignocellulosic plant fibres, fruit peelings, sugars, sunflower grain- or cotton hulls, yeast extract, peptone and/or whey protein) in a container and incubated in temperature-controlled conditions at 20-30 °C for 10 to 15 days. To prevent contamination, the container was sealed off with a filter-containing plastic cover or metal lid.

This fermentation process was adaptable to containers of various sizes, as the mycelium skin's size hinged on the liquid medium's available surface area, implying a theoretically limitless size for these skins. The liquid medium volume required for static surface fermentation culture correlated with the fermentation tray's area. In this study, a setup containing 3 L of liquid medium allowed growth on an approximately 0.15 m² surface area (0.5 m × 0.3 m), although 2 L of medium also yielded satisfactory mycelium mats. The use of 3 L of liquid medium ensured that a higher amount of nutrients was available for the same concentration. This anticipated the observation that, for the white rot species tested in this study, a sugar concentration exceeding 240 g/L negatively affected the growth rate and overall development of the mycelium. The mycelium mat was formed on the surface of the medium (FIG 2 and FIG 3), after which nutrient depletion occurred, resulting in a cessation of the organism's growth.

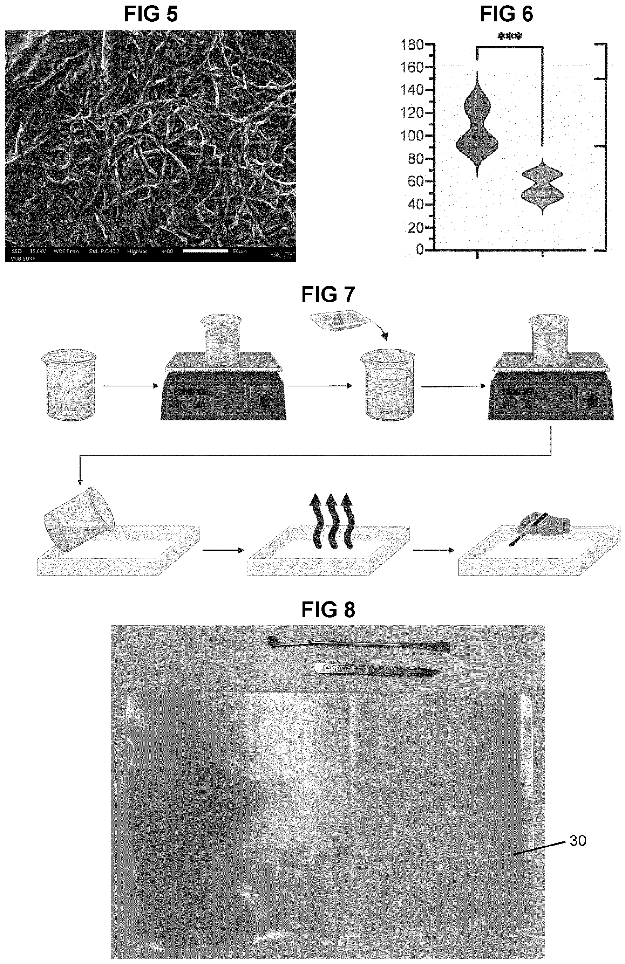

Following the incubation period, the skins were harvested from the liquid medium and washed with tap water to remove the polysaccharide layer formed between the mycelium skin and the medium. Subsequently, the skins were immersed in a treatment bath containing one or more of glycerol (10-25%) as plasticizing agent, a dyeing agent, binding agent (e.g. OrganoClick OC-BioBinder^{™} Lotus 54), CaCl₂ (1-2%) or tannins (1-5%), for a duration ranging from a few minutes to 72 hours (e.g. 5 minutes to 24 hours). The glycerol treatment imparted flexibility to the mycelium skins upon drying, while CaCl₂ and tannins could bestow antimicrobial properties on the prospective material (throughout its lifespan). Use of the binding agent (not to be confused with the binder mentioned below) as part of the post-treatment of the mycelium skins in particular significantly improved the properties of the mycelium skins, including their flexibility (i.e. akin to a plasticizing agent), hydrophobic properties, Taber abrasion resistance and wet surface abrasion resistance. Moreover, a binding agent such as OrganoClick OC-BioBinder^{™} Lotus 54 can additionally also function as a dyeing agent, with different shades of brown being available depending on the length (e.g. 1 hour to 100 hours) and temperature (e.g. 60-150 °C, preferably 80 to 100 °C) of curing (e.g. using an oven or heat press). Alternatively to the use of a treatment bath, the binding agent could also be applied in the form of a coating (e.g. applied using a paint brush or other suitable means). After a couple of minutes of drying, it was possible to an apply additoinal coating layer to increase the coating thickness; which could be repeated as needed in function of the desired coating thickness. In example of the hyphae of a mycellium skin being coated with a Lotus binding agent is shown in FIG 5.

Subsequently, the mycelium skins were taken out of the treatment bath and subjected to drying to reduce the moisture contained within the fungal tissue. The drying process could be executed in a well-ventilated space at room temperature (e.g. around 20 °C) or in a ventilated oven at 40-80 °C. The end result was plasticized dried mycelium skins (FIG 4), with properties that differed for the two sides: the aerial side had hydrophobic characteristics, while the side that was in contact with the liquid medium, was much more hydrophilic (FIG 6). It can be noted that depending on the fungal species used, the biomass yield, mycelial homogeneity, and extent of shrinking upon drying varied.

### Backing

To provide performant mechanical characteristics to the composite material, a plant- or animal-based mesh was used as a backing. The backing was internal and not visible in the final sandwich-structured material. Various woven or yarned fibre fabrics were used, such as hemp, cotton, jute, flax, ramie, kenaf, coir, bamboo, linen, wool or silk. The choice of backing could significantly impact the mechanical performance, as elaborated below when describing the mechanical characteristics of the final material. Moreover, the different backings contributed to varying thicknesses and bending characteristics of the final material.

### Binder

A gelatine-based thermoreversible hydrogel served the purpose of binder to glue the internal mesh backing to the external mycelium skins. Besides being an adhesive, it also served as a levelling agent that filled voids or uneven surfaces and it could provide additional mechanical properties to the material. Gelatine is a biopolymer and the other components could also be sourced biologically, so that the hydrogel could also be referred to as a bio-gel.

Before assembly into the mycelium-based composite material, flexible binder sheets were produced. By first creating a liquid hydrogel mixture and then casting this into a mould, the sheets could be manufactured in any preferred quantity, shape, or thickness (FIG 7). The hydrogel mixture included water, gelatine, and glycerol as main components, optionally in addition to a second biopolymer (e.g. agar), nanoparticles, a salt and/or crosslinking agent.

First, for instance 20 g gelatine was dissolved in a solution of 25 ml glycerol and-optionally-10 ml acetic acid in 200 ml tap water. This mixture was stirred with a magnetic stirrer at 300 rpm at a temperature of 45-60 °C for 2 to 3 hours. Optionally, the second polymeric ingredient (already dissolved) or other additives could be added to the gelatine/glycerol mixture followed by stirring this mixture with a magnetic stirrer at 500 rpm at 100 °C for 10 minutes. This mixture could then be poured into a mould, for example with dimensions of 20 × 280 × 470 mm and left to solidify and dry for 24-48 h at room temperature. Once the water was evaporated (and e.g. a constant weight was obtained), these sheets acquired a plastic-like material appearance with an approximate thickness of 0.1-3 mm (FIG 8). The composition of the hydrogel could vary in the concentrations used for each element; for example: 4-15% w/v gelatine, 4-10% v/v glycerol and 0-3% agar.

Glycerol functioned as a plasticizing agent due to its water-retaining properties, increasing the sheet's plasticity proportionate to the glycerol content. If the glycerol content was too low, the resulting material was stiff and brittle. Conversely, too much glycerol could result in a gummy-like texture, with excess glycerol oozing from the material and causing a greasy texture. Empirical testing of various glycerol and gelatine ratios suggested a general guideline for a good ratio of approximately 1:1, though 5:4 was mostly used.

While a second biopolymer like agar could optionally be added, excluding it from the mixture did not yield any discernible differences and was not a crucial ingredient to create a functional binder. To gain a better understanding of agar's effect on flexible gelatine sheets, a thermogravimetric analysis (TGA) was conducted to assess potential differences in thermal degradation profiles. The analysis indicated a higher mass loss for the sample containing 1.5% agar compared to one with just gelatine and glycerol. Due to this observation and the absence of clear advantages in adding agar, it was in most cases omitted from the binder preparation.

The initial liquid nature of the binder preparation allowed for an easy incorporation of other components (cf. supra) to bestow additional attributes to binder. For instance, acetic acid (e.g. 1-2% of pure acetic acid) could be added to the above-described gelatine/glycerol solution, with the aim of lowering the solution's pH, augmenting gelatine solubility, increasing the quantity of free amino acids, and ultimately elevating the polymer's mechanical strength. To render the binder water-insoluble and modify its mechanical properties, crosslinks between gelatine molecules could be introduced. This could be achieved through chemical means (e.g. using glutaraldehyde, genipin or a carbodiimide), enzymatic processes (e.g. using a transglutaminase, tyrosinase or horseradish peroxidase) or physical techniques (e.g. using a de-hydrothermal or ultraviolet radiation treatment) on gelatine as such and/or on a modified gelatinous substance, such as gelatine-norbornene, gelatine methacrylate or alginate methacrylate. The introduction of salts and/or nanoparticles could grant antimicrobial and/or antioxidant characteristics.

### Assembly

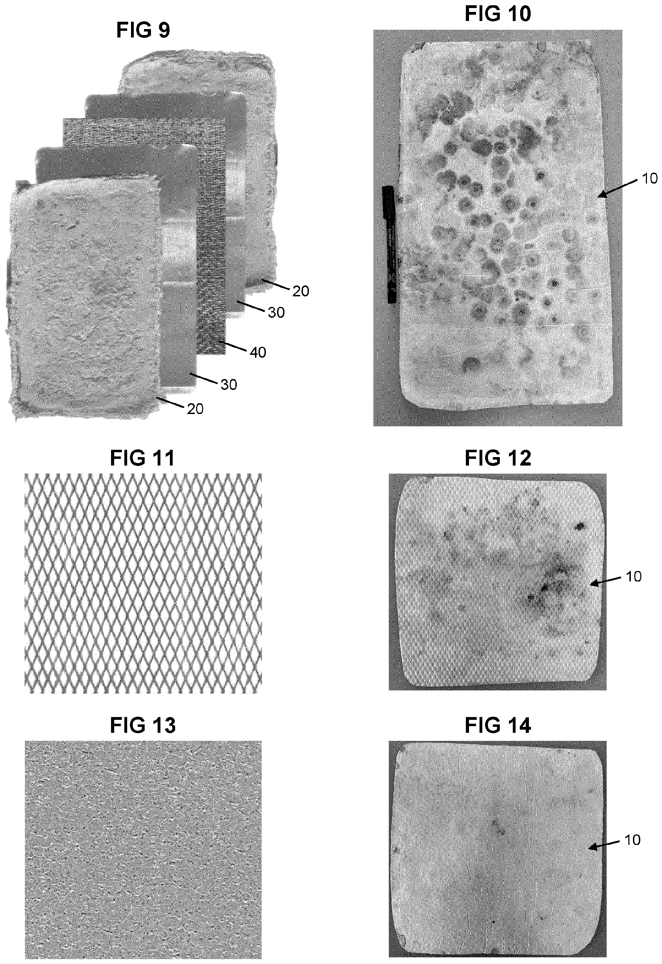

The assembly process involved creating a stack similar to a sandwich, where plasticized mycelium skins 20 were placed on the outer layers, the cellulosic fabric 40 backing occupied the centre position, and dried binder sheets 30 were inserted in between the internal fabric mesh 50 and the two outer mycelium layers 20 (FIG 9). The more hydrophobic aerial sides of the mycelium skins 20 were selected to face outwards.

Next, a heating and pressing procedure was performed using a heat press device, such as Ukpress professional heat press (Model HP3805B). This step was ideally conducted at temperatures ranging from 100 °C to 120 °C for a duration of 1 to 5 minutes (e.g. 60 to 90 s). The primary objective of this step was to melt the binder layer, facilitating the fusion of the exterior mycelium skins with the central backing element. The application of pressure served the dual purpose of combining these layers effectively and smoothing out the surface of the outer mycelium layer, addressing any inconsistencies in thickness that might be present across the skin's surface. For example, applying pressure on the stack helps to disperse the molten binder into the mycelium layer (and the backing). Upon heating, the thermoreversible gel binder sheet melted, subsequently resolidifying upon cooling to serve as a binding agent or glue between the mycelium skins and backing. The final material 10 resembled animal-based leather in appearance and empirically observed material characteristics (e.g. thickness and flexibility) (FIG 10).

Optionally, the composite material 10 could be provided with a texture by placing a patterned insert between the stack and the heat press. FIG 11 for instance shows a metal grid insert with a diamond pattern and FIG 12 shows the resulting embossed mycelium composite material 10, while FIG 13 shows a metal plate insert with a rough plaster relief and FIG 14 shows the corresponding embossed mycelium composite material 10.

### Characteristics

Prototype mycelium leather-like composite materials were characterized with various methodologies to assess their structural and mechanical attributes.

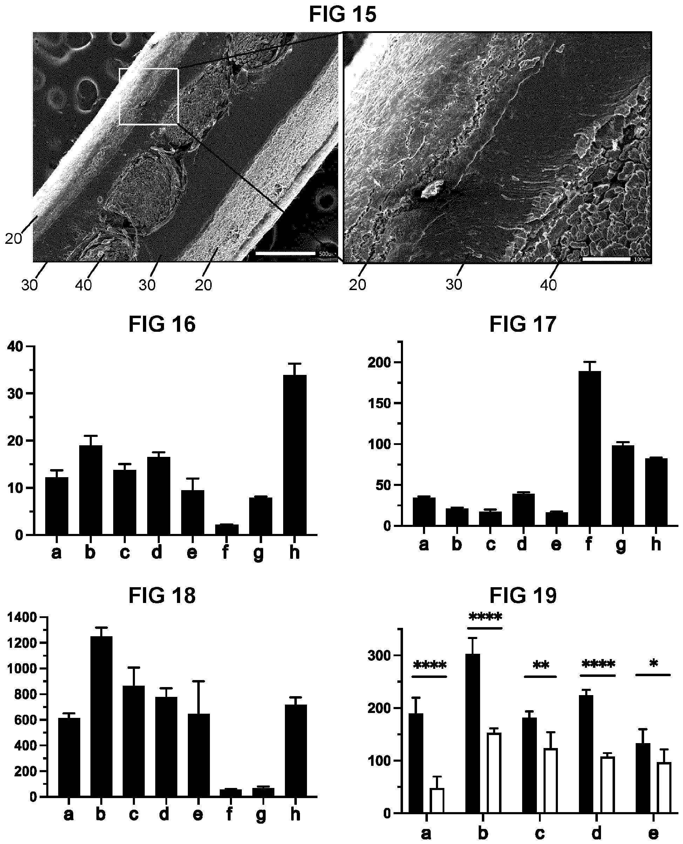

First, a microscopic analysis was performed on a cross-section of the composite material using electron microscopy (FIG 15), revealing a coherent material in which the binder layers attached to the fibres of the backing.

Different mycelium leather-like composites-including jute, fine linen, heavy linen, fine hemp and large hemp-were subjected to standard tensile testing in accordance with ISO 3376:2020 leather testing standards. These evaluations were conducted alongside three benchmark materials: polyurethane (PU), polyurethane vinyl (PUV) and cowhide leather. Tensile tests enabled the quantification of specific parameters like ultimate stress (FIG 16), strain (FIG 17), and Young's modulus (FIG 18) for the material. Results showed that composite materials with fine linen backing excelled with an average maximum shear force of 303.3 N, followed by those with fine hemp backing at 224.0 N. Composite materials with jute, heavy linen, and large hemp backing followed with 189.6 N, 181.2 N, and 133.5 N, respectively. These strengths outperformed or matched the synthetic benchmarks PU (16.35 N) and PUV (136.0 N), yet fell short of cowhide leather (620.25 N). The backing material predominantly determined shear strength, implying potential enhancement with stronger backing. Tightly woven fabrics exhibited robust tensile strength, influencing the mycelium leather-like material. Maximum stress and ultimate strain analyses aligned with these findings. The composite material samples had lower ultimate strain (16.63% to 39.30%) compared to synthetic (188.83% for PU, 97.98% for PUV) and cowhide leather (82.78%). Modulus results revealed unique behaviour: composite material with jute (614.13 Mpa) and large hemp (647.08 Mpa) backing showed slightly lower values than cowhide leather, while those with fine hemp (776.02 Mpa) and heavy linen (866.05 Mpa) backing surpassed it, and fine linen (1250.53 Mpa) exhibited the highest modulus.

Comparing the composite materials to the naked (standalone) backings as such, the combination of the gelatine binder layer with the backing and the mycelium layer increased the tensile strength (ultimate force) properties (FIG 19). Notably for backings with tightly woven fibres such as jute, fine hemp, and fine linen, this increase was denoted by a 4-star significance (P ≤ 0.0001). Conversely, backings with a more open weaving pattern, exemplified by heavy linen and large hemp, show a lower significance level of tensile strength increase, measured at 2-star (P ≤ 0.01) and 1-star (P ≤ 0.05), respectively. Remarkably, the jute backing experiences a substantial increase in tensile strength in the composite material, surging from 47.95 N to 189.7 N, marking a 3.95-fold increment. Similarly, the tensile strength of naked fine linen and fine hemp backings rose from 152.7 N to 303.3 N (an almost 2-fold increase) and 107.8 N to 224 N (more than 2-fold increase), respectively.

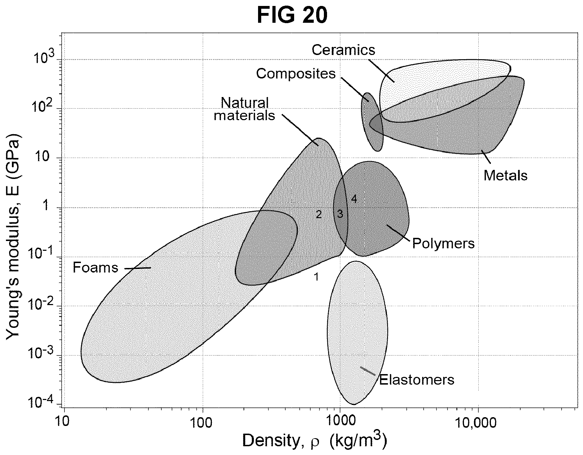

Mapping material density against Young's modulus allows for the positioning into a material chart, which serves to categorize various materials into distinct groups. This visual representation offers a simplified overview of a material's mechanical and physical attributes within the context of broader material families. As depicted in FIG 20, all mycelium leather-like composite samples are situated within the shared region of polymers and natural materials. However, an exception is noted for the fine linen samples, which exhibit a distinctive placement further within the polymer family and outside the collective classification of natural materials.

Thermogravimetric analysis (TGA) of the mycelium composite materials (not depicted) revealed that the binder is the primary source of mass loss during the initial stages of the thermal degradation process, peaking at approximately 250 °C. Subsequently, the mycelium undergoes thermal degradation, reaching its peak mass loss velocity just past 300 °C, followed by the linen backing, which experiences a similar peak, right before 400 °C. These findings suggest that improving the thermal resistance of the binder, potentially through the introduction of covalent crosslinks, can enhance the overall thermal stability of the mycelium composite material.

It is to be understood that although preferred embodiments, specific constructions, configurations and materials have been discussed herein in order to illustrate the present invention. It will be apparent to those skilled in the art that various changes or modifications in form and detail may be made without departing from the scope of the invention as defined in the appended claims.

## Claims

1. A composite material (10), comprising:
i) a layer of mycelium (20),
ii) a layer of binder (30), and
iii) a backing (40);
wherein the binder (30) comprises a thermoreversible gel.

2. The composite material (10) according to claim 1, wherein the thermoreversible gel is a hydrogel.

3. The composite material (10) according to any of the previous claims, wherein the thermoreversible gel comprises a gelatinous substance, preferably gelatine.

4. The composite material (10) according to any of the previous claims, wherein the thermoreversible gel has a gel temperature below 200 °C, preferably below 180 °C, more preferably below 150 °C, yet more preferably below 120 °C, still yet more preferably below 100 °C, most preferably below 80 °C.

5. The composite material (10) according to any of the previous claims, wherein the backing (40) is a mesh.

6. The composite material (10) according to any of the previous claims, wherein the binder (30) comprises a plasticizing agent, preferably glycerol.

7. The composite material (10) according to any of the previous claims, wherein the mycelium (20), binder (30) and/or backing (40) independently further comprises one or more additives.

8. The composite material (10) according to any of the previous claims, being a sandwich-structured composite material (10) comprising:
i) a first layer of mycelium (20),
ii) a first layer of binder (30),
iii) a backing (40),
iv) a second layer of binder (30), and
v) a second layer of mycelium (20).

9. A product comprising the composite material (10) according to any of the previous claims.

10. A method for forming a composite material (10), comprising:
a) providing a stack of
- a layer of mycelium (20), and
- either
a layer of binder (30) and
a backing (40)
or
a backing (40) infused with a binder (30); and
b) binding the mycelium (20) to the backing (40) using the binder (30).

11. The method according to claim 10, wherein step a comprises providing the layer of binder (30) as a solid layer.

12. The method according to claim 11, wherein step b comprises:
b1) heating the stack such that the binder (30) becomes fluid, and
b2) cooling the stack to re-gel the binder (30).

13. The method according to any of claims 10 to 12, wherein step b is performed while pressing the stack.

14. The method according to any of claims 10 to 13, for forming the composite material (10) as defined in claim 8, wherein step a comprises:
a) providing a stack of
- a first layer of mycelium (20),
- either
a first layer of binder (30),
a backing (40) and
a second layer of binder (30)
or
a backing (40) infused with a binder (30), and
- a second layer of mycelium (20).

15. Use of a layer of binder (30) in a composite material (10) as defined in any of claims 1 to 9, for binding the mycelium (20) to the backing (40).
